(19) **Europäisches Patentamt**
**European Patent Office**
**Office européen des brevets**

(11) **EP 2 053 066 A1**

(12) **EUROPEAN PATENT APPLICATION**

(43) Date of publication:
**29.04.2009 Bulletin 2009/18**

(51) Int Cl.:
*C08F 2/50* (2006.01)    *C07C 57/04* (2006.01)
*C07C 69/54* (2006.01)   *A61K 6/00* (2006.01)
*C07C 51/50* (2006.01)   *C07C 67/62* (2006.01)

(21) Application number: **09000633.9**

(22) Date of filing: **16.09.2005**

(84) Designated Contracting States:
**AT BE BG CH CY CZ DE DK EE ES FI FR GB GR HU IE IS IT LI LT LU LV MC NL PL PT RO SE SI SK TR**

(62) Document number(s) of the earlier application(s) in accordance with Art. 76 EPC:
**05020181.3 / 1 764 404**

(71) Applicant: **Straumann Holding AG**
**4002 Basel (CH)**

(72) Inventor: **Molenberg, Aaldert**
**4054 Basel (CH)**

(74) Representative: **Schaad, Balass, Menzl & Partner AG**
**Dufourstrasse 101**
**Postfach**
**8034 Zürich (CH)**

Remarks:
This application was filed on 17-01-2009 as a divisional application to the application mentioned under INID code 62.

(54) **Use of methylene blue as polymerization inhibitor**

(57) The present invention relates to a composition comprising a polymerizable compound with at least one conjugated unsaturated group and methylene blue to prevent premature polymerization of the polymerizable compound. Methylene blue in the present composition according to the present invention in a concentration of 10 to 5000 ppm.
The polymerizable compound is acrylic acid or an acrylic acid ester or a mixture thereof.

EP 2 053 066 A1

**Description**

The present invention relates to the prevention of premature polymerization during the transportation and storage of a polymerizable compound with at least one conjugated unsaturated group by addition of methylene blue as inhibitor.

Polymerizable compounds with at least one conjugated unsaturated group are widely used as basic chemicals. Examples are acrylic monomers, in particular acrylic acid and methacrylic acid. They are mostly used for the synthesis of polymers. In recent times such polymerizable compounds have been used in medicine.

Polymerizable compounds with at least one conjugated unsaturated group are also known to be used for forming biodegradable materials. In WO 01/92584 a matrix material is disclosed which is formed by nucleophilic addition reaction to conjugated unsaturated groups.

WO 00/44808 also discloses a polymeric biomaterial formed by nucleophilic addition reaction to conjugated unsaturated groups. The obtained hydrogels may be used for example as glues or sealants and as scaffolds for tissue engineering and wound healing applications.

During the manufacture, transport and storage of these polymerizable compounds with at least one conjugated unsaturated group, the so-called "runaway" polymerization has to be avoided, since this leads not only to quality loss of the monomer but also to safety problems. Especially when used in medicine such compounds have to fulfil purity requirements, since polymerization would impede stoichiometry of the nucleophilic addition reaction and cause premature gelation of a product. The research of past decades on the inhibition of polymerization of for example acrylic monomers has produced many usable inhibitors to prevent the radical polymerization.

An example a powerful inhibitor is a methyl ether of hydroquinone (MEHQ), which is usually present in acrylic acid at a concentration of approximately 200 ppm (S. Schulze, H. Vogel, Chem. Eng. Technol. 21, 829-836 (1998)). Said inhibitor works only in the presence of oxygen. Therefore MEHQ is not suitable for compositions which are to be used in medicine. Oxygen could impair the longterm stability of components of these compositions, e.g. peroxides could be formed in poly(ethylene glycol), and should therefore not be present in such compositions.

An other inhibitor which is industrially used to inhibit polymerization of said polymerizable compounds is phenothiazine (PTZ). In contrast to MEHQ, PTZ is also a good inhibitor in the absence of oxygen (Levy, J. Polymer Science: Part A: Polymer Chemistry, Vol 30, 569-576 (1992)). Said compound is known for its insecticidal, fungicidal, antibacterial and anthelmintic properties. Due to its widespread use in animals and man also many adverse reactions are known encompassing effects on blood elements, neuromuscular problems and photosensitization (Mitchell, S.C., the toxicity of phenothiazine, Drug Metabolism and Drug Interactions, 11, 204-235 (1994)). Due to said adverse reaction phenothiazine-based inhibitors should be replaced in compositions which are to be used in medicine.

Methylene blue is a well known dye. It is used as dye in medicine and is known to be biocompatible.

By "conjugated unsaturated group" the alternation of carbon-carbon, carbon-heteroatom or heteroatom-heteroatom multiple bonds with single bonds is meant.

By "polymerizable compound" is meant that said compound can undergo a polymerization reaction.

The problem of the present invention is to provide an inhibitor to prevent the polymerization of polymerizable compounds with at least one conjugated unsaturated group, which may be used in medicine.

The problem is solved by a composition according to claim 1. Further preferred embodiments are subject of claims 2 to 13.

Surprisingly it has been found that methylene blue has the ability to prevent the polymerization of polymerizable compounds with at least one conjugated unsaturated group. In the absence of oxygen methylene blue inhibits said polymerization reaction even better than the well known inhibitor phenothiazine. Methylene blue has to be present in a concentration of 10 to 5000 ppm, preferably 20 to 1000 ppm, to inhibit the polymerization of the polymerizable compounds over a long period of time. Surprisingly it could be shown that in absence of oxygen a composition comprising 100 ppm methylene blue is significantly better stabilized than a composition comprising 10 times more phenothiazine (1000 ppm).

Due to the fact that methylene blue has the ability to function as inhibitor in the absence of oxygen, said inhibitor may be used in all fields in which the presence of oxygen is undesired. As mentioned before, for example in pharmaceutical compositions the presence of oxygen should be avoided since oxygen could impair the stability of said compositions. Such compositions are generally in an inert atmosphere such as under argon or under nitrogen. Said compositions are oxygen-free or oxygen-poor, which means that the concentration of oxygen is less than 5%, preferably less than 2%, and most preferably less than 0.5%. It has been found that the absence of oxygen does not influence the activity of methylene blue as inhibitor of the polymerization reaction.

The blue colour of the composition according to the present invention has a positive side effect when used in medicine, since the visibility of the medicinal product is improved.

In a preferred embodiment the polymerizable compound with at least one conjugated unsaturated group is acrylic acid, methacrylic acid and esters thereof which are particularly liable to polymerize among vinyl compounds. Said compounds may be present alone or as mixtures. Examples of the acrylic ester include methyl acrylate, ethyl acrylate, butyl acrylate, 2-ethylhexyl acrylate, 2-hydroxyethyl acrylate, and 2-hydroxypropyl acrylate. Examples of the methacrylic ester include methyl methacrylate, butyl methacrylate, 2-hydroxyethyl methacrylate, and 2-hydroxypropyl methacrylate.

Polymerizable compounds which are used to form biodegradable matrix material are generally difficult to stabilize since they have to be stored under oxygen-free or oxygen-poor conditions and since the stabilizer has to be biocompatible. Said compounds are especially difficult to stabilize if they have a high number of short chains, and thus a high density of acrylates, resulting in a strong tendency to spontaneously polymerize. It has been found that said compounds which are described below are particularly well stabilized in the presence of methylene blue.

Polymerizable compounds which are used to form biodegradable matrix material comprise a core which carries 2 to 10 chains with a conjugated unsaturated group attached to any of the last 20 atoms of the chain. In a preferred embodiment said conjugated unsaturated group is terminal. The core can be a single atom such as a carbon or a nitrogen atom or small molecules such as an ethylene oxide unit, a sugar, a multifunctional alcohol, such as pentaerythritol or hexaglycerol. The chains are linear polymers or linear or branched alkyl chains optionally comprising heteroatoms, amide groups or ester groups. Beside the chains the core may be additionally substituted with linear or branched alkyl residues or polymers which have no conjugated unsaturated groups. In a preferred embodiment the compound has 2 to 10 chains, most preferably 4 to 8 chains.

The conjugated unsaturated group is preferably selected from the group consisting of acrylates, acrylamides, quinines, and 2- or 4-vinylpyridiniums.

In a most preferred embodiment the chains of the polymerizable compounds are linear polymers. Said polymers are preferably selected from the group consisting of poly(ethylene glycol), poly(vinyl alcohol), poly(ethylene-co-vinyl alcohol), poly(acrylic acid), poly(ethylene-co-vinyl-pyrrolidone), poly(ethyloxazoline), poly(vinyl pyrrolidone), poly(ethylene-co-vinyl pyrrolidone), poly(maleic acid), poly(ethylene-co-maleic acid), poly(acrylamide) or poly ethylene-co-poly (propylene oxide) block copolymers. Said polymers can also be copolymers, block copolymers, graft copolymers, or random copolymers. Blocks, which are polymerized on the ends of the hydrophilic polymers, can be composed of, for example, lactic acid, glycolic acid, ε-caprolactone, lactic-co-glycolic acid oligomers, trimethylene carbonate, anhydrides, and amino acids.

In a most preferred embodiment the chains of the polymerizable compounds are poly (ethylene glycol) molecules (PEG).

It has been found that methylene blue stabilizes particularly well an eight-arm branched PEG acrylate from hexaglycerol as shown below:

$$\text{Hexaglycerol } [- (CH_2CH_2O)_n\text{-}CH_2CH_2\text{-}O\text{-}CO\text{-}CH=CH_2]_8$$

wherein n is 2 to 200, preferably 3 to 10.

To obtain the stabilized composition according to the present invention the following procedure is preferred. Immediately after purification of the compound with at least one conjugated unsaturated group and before evaporation of the solvent, methylene blue is added to said compound. After mixing the components the solvent is evaporated which results in the stabilized composition according to the present invention.

The composition according to the present invention may additionally comprise ethanol which increases the solubility of methylene blue in the compound with at least one conjugated unsaturated group. To obtain such a composition ethanol is added after evaporation of the solvent.

The composition according to the present invention may be used in different fields. Especially preferred are fields in which the absence of oxygen and biocompatibility are important. Preferably the composition according to the present invention is used in medicine, in particular in dentistry.

**Example 1**

Samples of 8-arm PEG-acrylate 2k with different inhibitors were weighed into DSC pans and were heated until they had polymerized. Analysis was performed according to the ASTM698 method:
for each sample DSC traces (energy released vs. temperature) were recorded at different heating rates ($\beta$ = 4, 7, 10, and 13 K/min). The temperatures $T_{max}$ at which the polymerization peaks showed a maximum were recorded and the activation energy for the polymerization ($E_a$) was determined from eq. 1:

$$\ln \frac{\beta}{T_{max}^2} = const - \frac{E_a}{RT_{max}} \qquad (1)$$

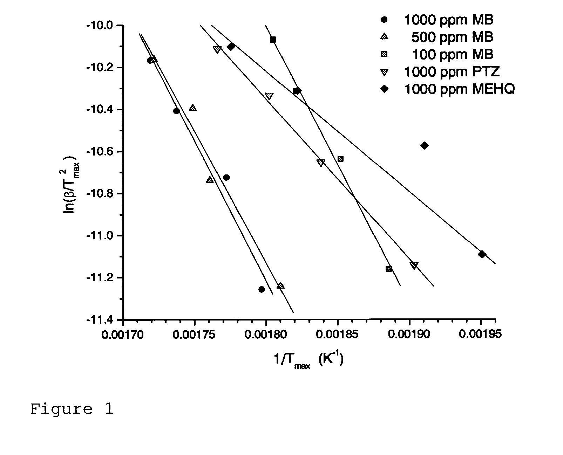

Figure 1 shows the data for 5 different samples.
The preexponential factor A was determined by eq. (2):

$$A = \frac{E_a}{RT_{max}^2} \beta \exp\left(\frac{E_a}{RT_{max}}\right) \tag{2}$$

The values of A were averaged over the four heating rates. The values of $E_a$ and average values of A are shown in table 1.

| Table 1 | | |
|---|---|---|
| | $E_a$ (kJ/mol) | A (min$^{-1}$) |
| 1000 ppm MB | 110 | $4.4 \cdot 10^9$ |
| 500 ppm MB | 110 | $1.3 \cdot 10^{10}$ |
| 100 ppm MB | 104 | $1.0 \cdot 10^9$ |
| 1000 ppm PTZ | 63 | $2.2 \cdot 10^5$ |
| 1000 ppm MEHQ | 48 | $6.4 \cdot 10^3$ |

By assuming the first order kinetics, ASTM698 permits evaluating the time ($t_\alpha$) to reach a certain extent of reaction ($\alpha$) as follows:

$$t_\alpha = \frac{-\ln(1-\alpha)}{A \exp\left(\frac{-E_a}{RT}\right)} \tag{3}$$

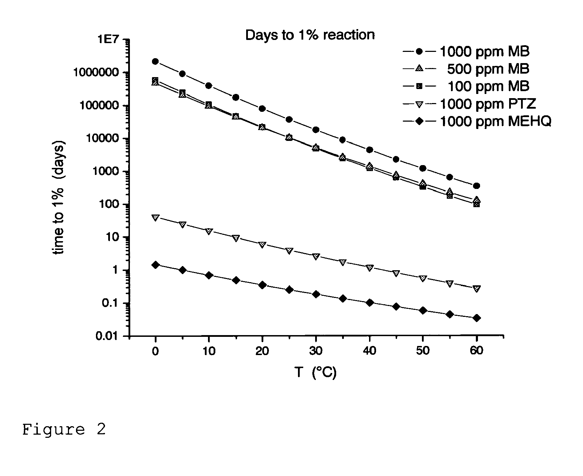

Using the experimentally evaluated values of $E_a$ and A, for each of the samples times to reach 1% of conversion ($\alpha = 0.01$ in eq. 3) at different temperatures T were estimated. The results are shown in Figure 2.

Figure 2 clearly shows the superior stability of the methylene blue stabilized samples compared to the PTZ and MEHQ stabilized samples.

**Example 2**

In order to establish a relationship between methylene blue (MB) concentration and stability of 8-arm PEG-acrylate 2k, a stress test has been performed.

Samples of one freshly synthesized 8-arm PEG-acrylate 2k batch (Nektar, batch# CM030703) were spiked with different concentrations of MB (250, 500, 1000, and 2000 ppm). For each MB concentration two 3 gram samples were added into separate 20 ml test tubes, which were closed with covers with argon inlets. The tubes were kept at 70°C under argon and the samples were checked daily until they had polymerized, as observed by solidification of the PEG oil.

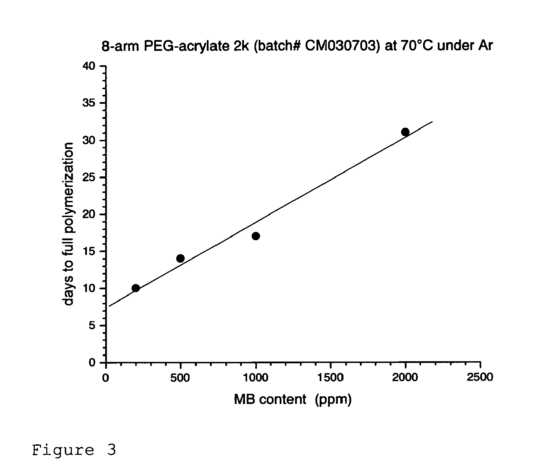

Figure 3 shows the results of the experiment. The linear relationship between MB content and stability confirms that the stabilizing effect is exerted by methylene blue.

**Example 3**

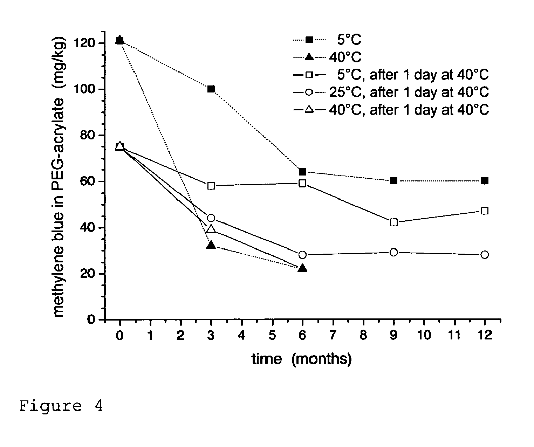

The content of methylene blue in 8-arm PEG-acrylate 2k was monitored in time under different simulated storage conditions for samples that were taken from the freezer and for samples that had been prestressed at 40°C for 1 day (figure 4). For all conditions the methylene blue content decreased in time, indicating that methylene blue was consumed while exerting its stabilizing function. After 6 months, methylene blue concentrations reached approximately constant values, which were higher for lower temperatures. The prestressed samples showed a clearly lowered methylene blue content at the start of the measurements, but levels of prestressed and non-prestressed samples became similar after some time at 40°C.

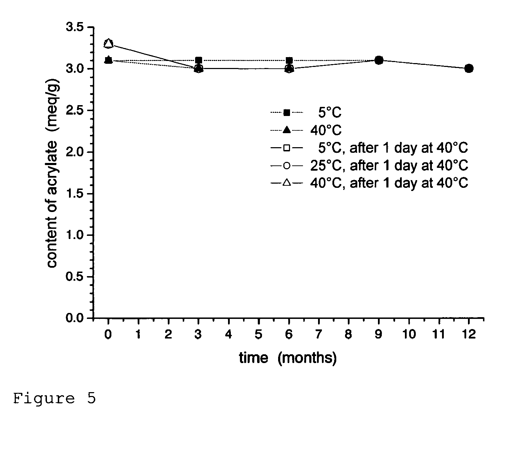

After 6 months at 40°C, some of the 8-arm PEG-acrylate 2k samples had partly or completely polymerized, indicating that a lower limit exists, under which the stabilizing effect of methylene blue is not given anymore. Figure 5 shows that the content of acrylate groups in the 8-arm PEG-acrylate 2k samples that had not polymerized remained constant over the whole observation period, which is another proof that the methylene blue present in the samples protects the acrylate groups from reacting. As becomes clear from figure 4, the effect of methylene blue is enhanced by choosing appropriately low storage temperatures.

The present invention further relates to a composition comprising a polymerizable compound with at least one conjugated unsaturated group and methylene blue to prevent premature polymerization of the polymerizable compound, characterized in that said composition contains methylene blue in a concentration of 10 to 5000 ppm.

In a preferred embodiment, said composition contains methylene blue in a concentration of 20 to 1000 ppm.

In a preferred embodiment, said composition comprises less than 5%, preferably less than 2% oxygen, more prefereably less than 0.5% oxygen.

In a preferred embodiment, the polymerizable compound is acrylic acid or an acrylic acid ester or a mixture thereof.

In a preferred embodiment, the polymerizable compound is methacrylic acid or a methacrylic acid ester or a mixture thereof.

In a preferred embodiment, the conjugated unsaturated group of the polymerizable compound is selected from the group consisting of acrylates, acrylamides, quinines, and 2- or 4 vinylpyridiniums.

In a preferred embodiment, the polymerizable compound comprises a core which carries 2 to 10 chains comprising said conjugated unsaturated groups, whereby said conjugated unsaturated groups are attached to any of the last 20 atoms of the chain.

In a preferred embodiment, the chains are poly(ethylene glycol) molecules.

In a preferred embodiment, the polymerizable compound is a 4 to 8-arm poly(ethylene glycol) acrylate.

The present invention also relates to the use of the composition according to the present invention in medicine.

The present invention also relates to the use of the composition according to the present invention in dentistry.

The present invention also relates to the use of 10 to 5000 ppm methylene blue in a composition comprising a polymerizable compound with at least one conjugated unsaturated group as inhibitor to prevent premature polymerization of the polymerizable compound.

## Claims

1. A composition comprising a polymerizable compound with at least one conjugated unsaturated group and methylene blue to prevent premature polymerization of the polymerizable compound, **characterized in that** said composition contains methylene blue in a concentration of 10 to 5000 ppm and that the polymerizable compound is acrylic acid or an acrylic acid ester or a mixture thereof.

2. A composition comprising a polymerizable compound with at least one conjugated unsaturated group and methylene blue to prevent premature polymerization of the polymerizable compound, **characterized in that** said composition contains methylene blue in a concentration of 10 to 5000 ppm and that the polymerizable compound is methacrylic acid or a methacrylic acid ester or a mixture thereof.

3. A composition according to claim 1 or 2,
   **characterized in that** said composition contains methylene blue in a concentration of 20 to 1000 ppm.

4. Composition according to any of the preceding claims comprising less than 5%, preferably less than 2% oxygen.

5. Composition according to any of the preceding claims comprising less than 0.5% oxygen.

6. Composition according to any of the preceding claims, wherein the polymerizable compound comprises a core which carries 2 to 10 chains comprising said conjugated unsaturated groups, whereby said conjugated unsaturated groups are attached to any of the last 20 atoms of the chain.

7. Composition according to claim 6, wherein the chains are poly(ethylene glycol) molecules.

8. Composition according to claim 1, wherein the polymerizable compound is a 4 to 8-arm poly(ethylene glycol) acrylate.

9. Use of the composition according to any of the preceding claims in medicine.

10. Use of the composition according to claims 1 to 8 in dentistry.

11. Use of 10 to 5000 ppm methylene blue in a composition comprising a polymerizable compound with at least one conjugated unsaturated group as inhibitor to prevent premature polymerization of the polymerizable compound, wherein the polymerizable compound is acrylic acid or an acrylic acid ester or a mixture thereof.

12. Use of 10 to 5000 ppm methylene blue in a composition comprising a polymerizable compound with at least one conjugated unsaturated group as inhibitor to prevent premature polymerization of the polymerizable compound, wherein the polymerizable compound is methacrylic acid or an methacrylic acid ester or a mixture thereof.

Figure 1

Figure 2

8-arm PEG-acrylate 2k (batch# CM030703) at 70°C under Ar

Figure 3

Figure 4

Figure 5

EP 2 053 066 A1

EUROPEAN SEARCH REPORT

Application Number

EP 09 00 0633

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DD 215 699 A (FRIEDRICH-SCHILLER-UNIVERSITAET JENA ,DD) 21 November 1984 (1984-11-21) * claims; example 2 * | 1,3-5, 9-11 | INV. C08F2/50 C07C57/04 C07C69/54 A61K6/00 C07C51/50 C07C67/62 |
| X | US 4 021 310 A (SHIMIZU ET AL) 3 May 1977 (1977-05-03) * claims; examples 8,16 * | 1,3-5,11 | |
| X | GB 942 318 A (IMPERIAL CHEMICAL INDUSTRIES LIMITED) 20 November 1963 (1963-11-20) * page 2, column 1, line 48 - line 51 * * page 2, column 2, line 73 - line 76 * | 2,3,12 | |
| X | DATABASE CA [Online] CHEMICAL ABSTRACTS SERVICE, COLUMBUS, OHIO, US; INSTITUTUL DE CERCETARI PRODUSE AUSILIARE ORGANICE, MEDIAS, ROM.: "Triethylene glycol dimethylacrylate" XP002366213 retrieved from STN Database accession no. 100:176196 * abstract * -& RO 81 794 A 30 May 1983 (1983-05-30) * column 2, paragraph 2; example 5 * | 2,3,11 | |
| A | PATENT ABSTRACTS OF JAPAN vol. 005, no. 152 (C-073), 25 September 1981 (1981-09-25) & JP 56 084708 A (MITSUBISHI GAS CHEM CO INC; others: 01), 10 July 1981 (1981-07-10) * abstract * | 1-12 | TECHNICAL FIELDS SEARCHED (IPC) C08F C07C A61K |

-/--

The present search report has been drawn up for all claims

| Place of search | Date of completion of the search | Examiner |
|---|---|---|
| Munich | 18 February 2009 | Österle, Carmen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

12

Europäisches
Patentamt

European
Patent Office

Office européen
des brevets

# EUROPEAN SEARCH REPORT

Application Number

EP 09 00 0633

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PATENT ABSTRACTS OF JAPAN vol. 1996, no. 10, 31 October 1996 (1996-10-31) & JP 08 155024 A (NIPPON ELECTRIC GLASS CO LTD), 18 June 1996 (1996-06-18) * abstract * | 1-12 | |
| D,A | L. LEVY: "Inhibition of acrylic acid polymerization by phenothiazine and p-methoxyphenol.II. Catalytic inhibition by phenothiazine" J.POLYMER SCIENCE: PART A: POLYMER CHEMISTRY, vol. 30, 1992, pages 569-576, XP009061210 * the whole document * | 1-12 | |
| D,A | H. VOGEL ET AL: "Aspects of the safe storage of acrylic monomers: Kinetics of the oxygen consumption" CHEM.ENG.TECHNOL., vol. 21, no. 10, 1998, pages 829-837, XP002366196 * the whole document * | 1-12 | |
| A | EP 0 334 500 A (UNIROYAL, INC) 27 September 1989 (1989-09-27) * the whole document * | 1-12 | TECHNICAL FIELDS SEARCHED (IPC) |

The present search report has been drawn up for all claims

| Place of search | Date of completion of the search | Examiner |
|---|---|---|
| Munich | 18 February 2009 | Österle, Carmen |

EPO FORM 1503 03.82 (P04C01)

## ANNEX TO THE EUROPEAN SEARCH REPORT
## ON EUROPEAN PATENT APPLICATION NO.

EP 09 00 0633

This annex lists the patent family members relating to the patent documents cited in the above-mentioned European search report.
The members are as contained in the European Patent Office EDP file on
The European Patent Office is in no way liable for these particulars which are merely given for the purpose of information.

18-02-2009

| Patent document cited in search report | | Publication date | Patent family member(s) | | Publication date |
|---|---|---|---|---|---|
| DD 215699 | A | 21-11-1984 | NONE | | |
| US 4021310 | A | 03-05-1977 | NONE | | |
| GB 942318 | A | 20-11-1963 | BE 605531 A1 | | 22-12-1961 |
| | | | NL 266444 A | | |
| | | | US 3210419 A | | 05-10-1965 |
| RO 81794 | A | 01-06-1983 | NONE | | |
| JP 56084708 | A | 10-07-1981 | JP 1240496 C | | 13-11-1984 |
| | | | JP 59018378 B | | 26-04-1984 |
| JP 08155024 | A | 18-06-1996 | NONE | | |
| EP 0334500 | A | 27-09-1989 | AR 246289 A1 | | 29-07-1994 |
| | | | AU 3157789 A | | 28-09-1989 |
| | | | BR 8901261 A | | 07-11-1989 |
| | | | CA 1335324 C | | 25-04-1995 |
| | | | DE 68900126 D1 | | 01-08-1991 |
| | | | JP 1278549 A | | 08-11-1989 |
| | | | JP 2103601 C | | 22-10-1996 |
| | | | JP 8019164 B | | 28-02-1996 |
| | | | MX 165348 B | | 05-11-1992 |
| | | | PT 90072 A | | 10-11-1989 |
| | | | RO 103126 B1 | | 12-05-1992 |
| | | | SU 1836383 A3 | | 23-08-1993 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0192584 A
- WO 0044808 A

**Non-patent literature cited in the description**

- **S. SCHULZE ; H. VOGEL.** *Chem. Eng. Technol.,* 1998, vol. 21, 829-836
- **LEVY.** *J. Polymer Science: Part A: Polymer Chemistry,* 1992, vol. 30, 569-576
- **MITCHELL, S.C.** the toxicity of phenothiazine. *Drug Metabolism and Drug Interactions,* 1994, vol. 11, 204-235